# EUROPÄISCHE PATENTANMELDUNG

(11) **EP 3 533 789 A1**
(43) Veröffentlichungstag der Anmeldung: **04.09.2019**
(21) Anmeldenummer: 18159120.7
(22) Anmeldetag: 28.02.2018
(51) Int. Cl.: C07D 251/34

(54) **VERFAHREN ZUR HERSTELLUNG VON TRIMETHALLYLISOCYANURAT**

(71) Anmelder: Evonik Degussa GmbH, 45128 Essen (DE)
(72) Erfinder: WERLE, Peter, 63571 Geinhausen (DE); BECK, Michael, 63512 Hainburg (DE); TRAGESER, Martin, 63571 Geinhausen-Höchst (DE)

(57) **Zusammenfassung**

Die vorliegende Erfindung richtet sich auf ein verbessertes Verfahren zur Herstellung von Trimethallylisocyanurat durch in situ Cu⁺-katalysierte Umsetzung von (Iso)cyanursäuresalzen mit Methallylhalogenid.

## Beschreibung

Die vorliegende Erfindung richtet sich auf ein verbessertes Verfahren zur Herstellung von Trimethallylisocyanurat durch in situ Cu⁺-katalysierte Umsetzung von (Iso)cyanursäuresalzen mit Methallylhalogenid.

### Hintergrund der Erfindung

Trimethallylisocyanurat (1,3,5-Tris-(2-methyl-propenyl)-1,3,5-triazin-2,4,6(1*H*,3*H*,5*H*)-trion; CAS-Nr. 6291-95-8; im Folgenden "TMAIC") ist ein trifunktionelles polymerisierbares Monomer, das Einsatz als Vernetzungskomponente für hochwertige Fluorkautschuke und Polyamide findet und auch als Katalysator bei der Laurinlactampolymerisation eingesetzt werden kann. Seine hervorragenden Eigenschaften liegen in der hohen thermischen Stabilität und der geringen Neigung zur Autopolymerisation.

Die Herstellung von TMAIC wird in der Literatur nach zwei Verfahrensweisen beschrieben. In einem Verfahren werden die Salze der (Iso)cyanursäure mit Methallylchlorid in dipolar aprotischen oder haloaromatischen Lösungsmitteln umgesetzt.

Beispielsweise beschreibt die GB 912,964 die Umsetzung von Trinatriumcyanurat (0.1 Mol) mit Methallylchlorid (0.6 Mol) in Dimethylformamid (DMF) bei 110 °C und 6 h Reaktionszeit mit anschließender Destillation des DMF. Die Aufarbeitung des Rückstandes ergibt TMAIC mit einer Ausbeute von 70.2 %. Dies wird zur weiteren Reinigung aus wässrigem Ethanol umkristallisiert. Dementsprechend arbeitet das Verfahren der US 3,376,301 in Dimethylformamid.

Die US 3,075,979 beschreibt ein Verfahren, in dem Cyanursäure, Triethylamin und Methallylchlorid in *ortho*-Dichlorobenzol umgesetzt werden. Die Gesamtreaktionszeit beträgt 11 Stunden, in denen die Reaktionstemperatur von 110 °C auf 160 °C ansteigt. Nach Abtrennung des gebildeten Triethylaminhydrochlorids und Destillation des Lösungsmittels verbleibt das TMAIC in einer Ausbeute von 89 %.

Das andere in der Literatur beschriebene Verfahren ist die Umsetzung von Alkalicyanaten (vorwiegend Natriumcyanat, NaOCN) mit Methallylchlorid in DMF. Dieses Verfahren ist beispielsweise beschrieben in der JP 2000-109314 A. In dem darin beschriebenen Verfahren wird NaOCN in einer Vorstufe aus Natriumcarbonat und Harnstoff in situ hergestellt und danach die Umsetzung mit Methallylchlorid bei 140 °C für 2 Stunden durchgeführt. Nach Aussage der JP 2000-109314 A wird TMAIC danach in einer Ausbeute von 92 % erhalten. Die Erfinder der vorliegenden Erfindung konnten dies jedoch nicht bestätigen: Bei der Reproduktion des entsprechenden Versuchs aus der JP 2000-109314 A konnte TMAIC lediglich mit 10 % Ausbeute erhalten werden.

Auch beim Einsatz von kommerziell erhältlichem Natrium-oder Kaliumcyanat lassen sich nur mäßige Ausbeuten erhalten, die etwa 60 % der Theorie erreichen. Selbst nach mehrmaligen Umkristallisationen verbleibt außerdem ein unangenehmer, aminartiger Geruch. Vermutlich liegt dies daran, dass das Verfahren von JP 2000-109314 A hinsichtlich der Synthese von Triallylcyanurat optimiert wurde, aber nicht ohne weiteres auf die Synthese von TMAIC übertragbar ist.

Dies gilt auch für einen anderen Prozess, der bei der Herstellung von Triallylisocyanurat technisch durchgeführt wird (US 3,065,231; T.C. Frazier, E.D. Little, B.E. Lloyd, J. Org. Chem. 1960, 25, 1944 - 1946). Es handelt sich dabei um die Umsetzung von Allylchlorid mit dem Natriumsalz der Cyanursäure in wässriger Lösung, wobei Kupfer(I)-Chlorid als Katalysator fungiert. Wenn man dieses Verfahren auf die Herstellung von TMAIC überträgt und statt Allylchlorid Methallylchlorid einsetzt, werden nur Ausbeuten von 21 % an der Zielverbindung erreicht.

Die Aufgabe der vorliegenden Erfindung bestand demnach darin, ein verbessertes Verfahren zur Herstellung von TMAIC zur Verfügung zu stellen, welches die vorgenannten Nachteile nicht aufweist. Insbesondere sollte dieses Verfahren eine gegenüber den Verfahren des Standes der Technik verbesserte Ausbeute an TMAIC ermöglichen.

Es wurde nun ein Verfahren gefunden, welches die erfindungsgemäße Aufgabe löst.

### Detaillierte Beschreibung der Erfindung

Die vorliegende Erfindung betrifft ein Verfahren zur Herstellung von TMAIC, in welchem
(a) mindestens ein Methallylhalogenid, insbesondere Methallylchlorid oder Methallylbromid, bevorzugt Methallylchlorid und
(b) mindestens ein Salz der (Iso)cyanursäure, insbesondere ein Trialkalimetallsalz der (Iso)cyanursäure, bevorzugt Trinatriumcyanurat,
in Gegenwart eines Katalysatorsystems **K** zu TMAIC umgesetzt werden,
welches dadurch gekennzeichnet ist, dass das Katalysatorsystem **K** eine Kombination aus einem Kupfer(II)-Salz und einem Reduktionsmittel **R,** welches Kupfer(II) in situ zu Kupfer(I) reduziert, umfasst.

Es wurde überraschend gefunden, dass dadurch eine im Vergleich zu den Verfahren des Standes der Technik verbesserte Ausbeute an TMAIC erhalten wird.

Das erfindungsgemäße Verfahren kann in den dem Fachmann bekannten Vorrichtungen (beschrieben im Stand der Technik, z.B. US 3,065,231) und batchweise oder kontinuierlich durchgeführt werden.

(Iso)cyanursäure bedeutet im Sinne der Erfindung "Cyanursäure oder Isocyanursäure", bevorzugt "Cyanursäure".

In dem Verfahren werden zwei Edukte eingesetzt.

Zum Einen wird mindestens ein Methallylhalogenid eingesetzt. Grundsätzlich können dazu alle Methallylhalogenide, also Methallylfluorid, Methallylchlorid, Methallylbromid oder Methallyljodid verwendet werden. Bevorzugt ist das Methalyllhalogenid jedoch ausgewählt aus Methallylchlorid, Methallylbromid. Am bevorzugtesten ist es Methallylchlorid.

Das andere Edukt ist mindestens ein Salz der (Iso)cyanursäure. Bevorzugt wird dabei ein Trialkalimetallsalz der (Iso)cyanursäure, bevorzugt Trinatrium(iso)cyanurat, eingesetzt.

Dieses Salz der (Iso)cyanursäure kann auch in situ gebildet werden, in dem zum Beispiel Isocyanursäure oder Cyanursäure zusammen mit einem Hydroxid des entsprechenden Metalls vorgelegt werden. Dieses Vorgehen ist beispielsweise dann bevorzugt, wenn ein Trialkalimetallsalz der (Iso)cyanursäure eingesetzt wird. Dann werden die Cyanursäure oder die Isocyanursäure mit der entsprechenden Menge, bevorzugt mindestens 3 Mol-Äquivalente, des Alkalimetallhydroxids vorgelegt, und es bildet sich das Alkalimetallsalz der (Iso)cyanursäure.

Das erfindungsgemäße Verfahren wird mit oder ohne Lösungsmittel durchgeführt. So kann beispielsweise das Edukt Methallylchlorid zur Lösung bzw. Aufschlämmung der Reaktanden genutzt werden.

Bevorzugt wird das erfindungsgemäße Verfahren, also die Umsetzung des mindestens einen Methallylhalogenids mit dem mindestens einen Salz der (Iso)cyanursäure in Gegenwart eines Katalysatorsystems **K** zu TMAIC in wässriger Lösung durchgeführt.

Erfindungswesentlich ist, dass die Edukte Methallylhalogenid und das Salz der (Iso)cyanursäure in Gegenwart eines Katalysatorsystems **K** umgesetzt werden.

Das Katalysatorsystem **K** umfasst zwei Komponenten.

Zum einen ein Kupfer(II)-Salz. Das Kupfer(II)-Salz ist dabei nicht weiter beschränkt. Bevorzugt werden jedoch Kupfer(II)-Salze ausgewählt aus der Gruppe bestehend aus Kupfer(II)chlorid, Kupfer(II)bromid, Kupfer(II)sulfat, Kupfer(II)acetat, Kupfer(II)nitrat eingesetzt. Diese sind wasserlöslich sind, was gerade in den Fällen, in denen Wasser als Lösungsmittel eingesetzt wird, von Vorteil und deshalb bevorzugt ist.

Das Reduktionsmittel **R** ist die zweite Komponente des Katalysatorsystems **K.** Erfindungswesentlich ist, dass das Reduktionsmittel **R** das Kupfer(II)-Salz in situ zu Kupfer(I) reduzieren kann. Ein solches Reduktionsmittel, was diese Voraussetzung erfüllt, ist vom Fachmann anhand des Standardpotentials des jeweiligen Redoxpaares leicht zu bestimmen. Die Standardpotentiale einiger Redoxpaare und damit die Wahl eines für das Redoxpaar Cu(II)/Cu(I) geeignetes Reduktionsmittel ergeben sich aus der elektrochemischen Spannungsreihe, wie sie beispielsweise auf Seite 159 des Lehrbuches "Chemie heute; Sekundarbereich II, Schroedel Schulbuchverlag, 1988, ISBN 3-507-10618-3" beschrieben sind.

Bevorzugte Reduktionsmittel **R** sind ausgewählt aus der Gruppe bestehend aus
- den Säuren des Schwefels, in denen der Schwefel nicht vollständig oxidiert ist, bevorzugt schweflige Säure, Sulfoxylsäure, dithionige Säure, dischweflige Säure, und deren Salze, insbesondere die Alkalimetallsalze, insbesondere Natriumsalze, der genannten Sauerstoffsäuren des Schwefels,
- Säuren des Phosphors, in denen der Phosphor nicht vollständig oxidiert ist, bevorzugt Phosphonsäure, Phosphinsäure und deren Salze, insbesondere die Alkalimetallsalze, insbesondere Natriumsalze, der genannten Säuren des Phosphors,
- Hydrazin, Hydroxylamin und deren Salze, wobei bevorzugte Salze Hydrazinsulfat, Bis(hydroxylammonium)sulfat, Hydroxylammoniumchlorid,
- Natriumborhydrid.

Besonders bevorzugt sind die genannten Säuren des Schwefels und deren Salze, insbesondere Natriumdithionit, und Hydrazin.

Im Katalysatorsystem **K** liegt das molare Verhältnis des Kupfer(II)-Salzes zum Reduktionsmittel **R** bevorzugt im Bereich 1 : 1 bis 1 : 2, bevorzugt 1 : 1 bis 1 : 1.1, noch bevorzugter 1 : 1 bis 1 : 1.075.

Im erfindungsgemäßen Verfahren werden die Edukte Methallylhalogenid und das Salz der (Iso)cyanursäure in Gegenwart des Katalysatorsystems **K** umgesetzt. Diese Umsetzung wird bevorzugt in wässriger Lösung durchgeführt.

Die Temperatur ist dabei nicht weiter beschränkt, es wird jedoch bevorzugt bei einer Temperatur von 20 °C bis 95 °C, noch bevorzugter 30 °C bis 80 °C, noch bevorzugter 40 °C bis 70 °C, noch bevorzugter 50 °C bis 65 °C, am bevorzugtesten bei 60 °C bis 65 °C durchgeführt.

Es hat sich außerdem als vorteilhaft erwiesen, und es ist deshalb bevorzugt, dass in den Fällen, in denen das erfindungsgemäße Verfahren in wässriger Lösung durchgeführt wird, während der Umsetzung ein pH-Wert im alkalischen Bereich, also > pH 7, eingestellt wird. Bevorzugt ist dann ein pH-Wert im Bereich 7.5 bis 13, bevorzugter 8 bis 12, noch bevorzugter 9 bis 11, am bevorzugtesten 10 bis 10.5. Der pH-Wert wird bevorzugt mit NaOH eingestellt, insbesondere wenn als das Salz der (Iso)cyanursäure ein Natriumsalz ist.

Die Umsetzung der Edukte in Gegenwart des Katalysators kann vom Fachmann routinemäßig, z.B. mit Chromatographie, verfolgt werden. Die Reaktionszeit kann dementsprechend bis zur vollständigen Umsetzung eingestellt werden. Sie beträgt bevorzugt nicht mehr als 2 Stunden, noch bevorzugter 15 bis 90 Minuten, noch mehr bevorzugter 25 bis 60 Minuten.

Das Verhältnis der Edukte ist nicht weiter beschränkt. Schon aus der Stöchiometrie der Reaktion ergibt sich aber, dass die Edukte Methallylhalogenid und das Salz der (Iso)cyanursäure bevorzugt in solch einem molaren Verhältnis eingesetzt werden, dass das molare Verhältnis aus der Gesamtmenge an eingesetztem Methallylhalogenid zur Gesamtmenge an allen eingesetzten Salzen der (Iso)cyanursäure ≥ 3 : 1 ist, noch bevorzugter im Bereich 3 : 1 bis 12 : 1, noch mehr bevorzugter bei 9 : 1 liegt.

Ein Überschuss von mehr als 3 : 1 hat sich als vorteilhaft erwiesen, da das überschüssige Methallylhalogenid als Lösungsmittel für das gebildete TMAIC dient und eine saubere Trennung des sich nach dem Ende der Reaktion ausbildenden zweiphasigen Reaktionsgemisches ermöglicht. So kann das in der wässrigen Phase verbleibende Cu(I) praktisch quantitativ von der oberen rein organischen Phase separiert werden.

Die Aufarbeitung erfolgt im Übrigen routinemäßig. Nach Ende der Reaktion wird die Produktphase mit Wasser gewaschen, die Phasen getrennt und das überschüssige Methallylhalogenid abdestilliert. Der TMAIC- Rückstand kann dann mit einem geeigneten organischen Lösungsmittel versetzt werden. Dieses hat vorteilhafterweise die Eigenschaft, sich bei erhöhten Temperaturen (80 °C) mit TMAIC zu mischen und dieses bei niedrigerer Temperatur wieder abzuscheiden. Geeignet sind hier bevorzugt niedere aliphatische Alkohole mit bevorzugt 1 bis 6 Kohlenstoffatomen, am bevorzugtesten n-Propanol. Das TMAIC wird dann mit Hilfe dieses Alkohols wie beschrieben umkristallisiert.

Alternativ ist eine Reinigung des nach Abdestillieren des Lösungsmittels erhaltenen Roh-TMAIC auch über eine Destillation im Hochvakuum möglich.

Die folgenden Beispiele sollen die Erfindung näher erläutern, ohne diese einzuschränken.

### Beispiele

### Erfindungsgemäßes Beispiel E1

194 g (1.5 Mol) Isocyanursäure, 180 g (4.5 Mol) NaOH und 7.5 g (43 mMol) Natriumdithionit (Na₂S₂O₄) wurden in 2 I Wasser eingetragen und unter Rühren gelöst. Dann wurden 832 g 98%-iges Methallylchlorid (9.0 Mol) zugegeben und die Reaktion durch Zugabe einer Lösung von 6 g (40 mMol) CuCl₂ in 15 ml Wasser gestartet. Der anfängliche pH-Wert der Lösung nahm nach Beginn der Reaktion ab und wurde durch Zugabe von Natronlauge im Bereich 10 bis 10.5 gehalten. Die Temperatur stieg auf ∼ 60 °C an und wurde dort durch Wärmezufuhr für etwa 20 Minuten belassen. Nach Ende der Reaktion wurde durch Zusatz von Mineralsäure ein pH von 2 eingestellt. Die wässrige Phase wurde abgetrennt und die Reaktionslösung mit 1 I Wasser gewaschen. Nach Abtrennung der wässrigen Phase wurde das Methallylchlorid aus der TMAIChaltigen Phase bei 90 °C und 18 hPa entfernt. Der Rückstand wurde unter Rühren mit 410 ml *n-*Propanol versetzt, bei 85 °C homogenisiert und filtriert. Danach wurde auf 5 °C gekühlt und das ausgefallene TMAIC abgesaugt, mit wenig *n*-Propanol gewaschen und getrocknet. Es wurden 408 g TMAIC (93.5 %) erhalten.

### Erfindungsgemäßes Beispiel E2

Das Beispiel **E1** wurde wiederholt, nur dass man statt Natriumdithionit 3.6 g Hydrazinhydrat zusetzte. Es wurden 406 g (93 %) TMAIC erhalten.

### Ergebnis

Die erfindungsgemäßen Beispiele zeigen, dass sich das erfindungsgemäße Verfahren hervorragend zur Herstellung von TMAIC eignet. Insbesondere ist die gewonnene Ausbeute von 93 % oder mehr besser als die, die für die Verfahren des Standes der Technik beschrieben wurde und erst recht als die, die bei Nachstellung dieses Verfahrens für TMAIC erhalten wurde.

Es war nicht naheliegend, dass das in der US 3,065,231 und von T.C. Frazier, E.D. Little, B.E. Lloyd J.Org.Chem. 1960. 25, 1944-1946 beschriebene Verfahren zur Herstellung von TAIC durch den erfindungsgemäßen Einsatz eines Reduktionsmittels so modifiziert werden konnte, dass es sich zur Herstellung von TMAIC mit solch guten Ausbeuten eignet.

Die JP 2000-109314 A legte dies erst recht nicht nah, da diese einen völlig anderen Reaktionsweg beschreibt.

## Patentansprüche

1. Verfahren zur Herstellung von Trimethallylisocyanurat, in welchem
(a) mindestens ein Methallylhalogenid und
(b) mindestens ein Salz der (Iso)cyanursäure,
in Gegenwart eines Katalysatorsystems **K** zu Trimethallylisocyanurat umgesetzt werden,
welches **dadurch gekennzeichnet ist,**
**dass** das Katalysatorsystem **K** eine Kombination aus einem Kupfer(II)-Salz und einem Reduktionsmittel **R,** welches Kupfer(II) in situ zu Kupfer(I) reduziert, umfasst.

2. Verfahren nach Anspruch 1, wobei das Methallylhalogenid ausgewählt ist aus Methallylbromid, Methallylchlorid.

3. Verfahren nach Anspruch 1 oder 2, wobei das Salz der (Iso)cyanursäure ein Trialkalimetallsalz der (Iso)cyanursäure ist.

4. Verfahren nach einem der Ansprüche 1 bis 3, welches in wässriger Lösung durchgeführt wird.

5. Verfahren nach einem der Ansprüche 1 bis 4, wobei das Kupfer(II)-Salz ausgewählt aus der Gruppe bestehend aus Kupfer(II)chlorid, Kupfer(II)bromid, Kupfer(II)sulfat, Kupfer(II)acetat, Kupfer(II)nitrat ist.

6. Verfahren nach einem der Ansprüche 1 bis 5, wobei das Reduktionsmittel ausgewählt aus der Gruppe bestehend aus
- den Säuren des Schwefels, in denen der Schwefel nicht vollständig oxidiert ist, und deren Salze,
- den Säuren des Phosphors, in denen der Phosphor nicht vollständig oxidiert ist, und deren Salze,
- Hydrazin und dessen Salze,
- Hydroxylamin und dessen Salze,
- Natriumborhydrid
ist.

7. Verfahren nach einem der Ansprüche 1 bis 6, welches bei einer Temperatur von 20 °C bis 95 °C durchgeführt wird.

8. Verfahren nach einem der Ansprüche 1 bis 7, welches in wässriger Lösung durchgeführt wird und wobei während der Umsetzung ein pH-Wert im alkalischen Bereich eingestellt wird.

9. Verfahren nach einem der Ansprüche 1 bis 8, wobei die Edukte Methallylhalogenid und das Salz der (Iso)cyanursäure in solch einem molaren Verhältnis eingesetzt werden, dass das molare Verhältnis aus der Gesamtmenge an eingesetztem Methallylhalogenid zur Gesamtmenge an allen eingesetzten Salzen der (Iso)cyanursäure ≥ 3 : 1 ist.

10. Verfahren nach einem der Ansprüche 1 bis 9, welches kontinuierlich durchgeführt wird.
